# EUROPEAN PATENT APPLICATION

(11) **EP 3 991 800 A1**
(43) Date of publication of application: **04.05.2022**
(21) Application number: 21204891.2
(22) Date of filing: 27.10.2021
(51) Int. Cl.: A61Q 19/00, A61K 8/02, A61K 8/9789

(54) **GINSENG POWDER, COSMETIC COMPOSITION COMPRISING SAME AND PREPARATION METHOD THEREOF**

(30) Priority: 28.10.2020 KR 20200141309
(71) Applicant: Amorepacific Corporation, Seoul 04386 (KR)
(72) Inventor: WOO, Byoung Young, Yongin-si Gyeonggi-do 17074 (KR); KIM, Hyungmin, Yongin-si Gyeonggi-do 17074 (KR); PARK, Sejun, Yongin-si Gyeonggi-do 17074 (KR); LEE, Somi, Yongin-si Gyeonggi-do 17074 (KR); HONG, Yong Deog, Yongin-si Gyeonggi-do 17074 (KR)
(74) Representative: Germain Maureau

(57) **Abstract**

The present disclosure relates to a ginseng powder, a cosmetic composition containing the same and a method for preparing the same. The present disclosure provides a ginseng powder with superior dissolution rate of active ingredients even at relatively low temperature. Therefore, the present disclosure provides an effect that the active ingredients contained in ginseng can be utilized effectively.

## Description

### BACKGROUND

### 1. Field

The present disclosure discloses a ginseng powder, a cosmetic composition comprising the same and a method for preparing the same.

### 2. Description of the Related Art

Ginseng is generally classified into fresh ginseng, white ginseng and red ginseng depending on its state. Fresh ginseng refers to a ginseng which has not been dried after being harvested and contains about 75 wt% of water, white ginseng refers to a ginseng which has been obtained by peeling and drying fresh ginseng, and red ginseng refers to a ginseng which has been obtained by steaming and drying fresh ginseng without peeling. Meanwhile, a residue remaining after extracting active ingredients contained in white ginseng or red ginseng is called a ginseng residue. The ginseng residue is discarded even though it is expected to have various physiological activities since it contains polysaccharides. Although Korean Patent Registration Publication No. 10-0385626 presents a method of utilizing the ginseng residue, it merely describes use as a feed additive and industrial application is very difficult. Accordingly, it is necessary to develop a technology which allows effective utilization of the active ingredients contained in ginseng either by preventing the production of a ginseng residue or by utilizing the ginseng residue.

### SUMMARY

The present disclosure is directed to providing a ginseng powder, a cosmetic composition comprising the same and a method for preparing the same, which allow effective utilization of extracted ingredients of ginseng and active ingredients comprised in ginseng.

In an aspect, the present disclosure provides a ginseng powder comprising particles, wherein the particles have a D90 less than 16 µm, and wherein the D90 is according to a volume-based particle diameter distribution measured by laser diffractometry.

In another aspect, the present disclosure provides a cosmetic composition comprising the ginseng powder.

In another aspect, the present disclosure provides a method for preparing a ginseng powder, comprising: a) obtaining a first ginseng powder by coarsely grinding a tissue-softened ginseng; and b) obtaining a second ginseng powder by grinding the first ginseng powder.

The present disclosure provides a ginseng powder with superior dissolution rate of active ingredients even at relatively low temperature. Therefore, the active ingredients comprised in ginseng can be utilized effectively.

### BRIEF DESCRIPTION OF THE DRAWINGS

FIG. 1 shows the particle shape of ginseng powders according to an example and a comparative example of the present disclosure.
FIG. 2 shows the particle size distribution of ginseng powders according to comparative example.
FIG. 3 shows the particle size distribution of ginseng powders according to the present disclosure.
FIG. 4 shows the dissolution rate of ginseng powders according to an example and comparative examples of the present disclosure.
FIG. 5 shows the desorption electrospray ionization mass spectrometry (DESI-MS) analysis result of a ginseng powder.

### DETAILED DESCRIPTION

The terms used in the present specification are selected from among general terms that are currently widely used in consideration of their functions in the present disclosure. However, the terms may be different according to the intent of one of ordinary skill in the art, precedents, the advent of new technology, etc. In addition, in particular cases, the terms are discretionally selected by the applicant, and the meaning of those terms will be described in detail in the corresponding part of the detailed description. Therefore, the terms used in the present disclosure should be defined based on not the simple designations of the terms but the meaning of the terms and the context of the present disclosure.

Unless defined otherwise, all the terms including technical or scientific terms used herein have the same meanings as commonly understood by those skilled in the art. The generally understood terms should be interpreted as having meanings consistent with the meanings in the context of related technologies, and should not be construed in an ideal or excessively formal sense, unless explicitly defined in the present disclosure.

All numerical ranges are inclusive of narrower ranges; delineated upper and lower range limits are interchangeable to create further ranges not explicitly.

Hereinafter, the present disclosure will be described in detail referring to examples and drawings. However, it is obvious that the present disclosure is not limited by the examples and drawings.

Laser diffractometry is a method of determining the size distribution or diameter distribution of particles. The "Dx according to a volume-based particle diameter distribution" determined by the measurement method refers to a diameter corresponding to a cumulative volume percentage x% in a volume-based particle size distribution chart. The Dx may be also expressed by [Dₓ], D_{[x]}, [Dx], D(0.x), D[0.x], etc.

Accordingly, D50 according to a volume-based particle diameter distribution refers to a diameter corresponding to a cumulative volume percentage 50% in a volume-based particle size distribution chart. Likewise, D90 according to a volume-based particle diameter distribution refers to a diameter corresponding to a cumulative volume percentage 90% in a volume-based particle size distribution chart.

In an aspect, the present disclosure provides a ginseng powder comprising particles, wherein the particles have a D90 less than 16 µm, and wherein the D90 is according to a volume-based particle diameter distribution measured by laser diffractometry. Specifically, the D90 may be more than 5 µm, 5.5 µm or more, 5.7 µm or more, 6 µm or more, 6.5 µm or more, 7 µm or more, 7.5 µm or more, 8 µm or more, 8.5 µm or more, 9 µm or more, 9.5 µm or more, 10 µm or more, 10.1 µm or more, 10.5 µm or more, 11 µm or more, 11.5 µm or more, 12 µm or more or 12.4 µm or more; and less than 16 µm, 15.6 µm or less, 15 µm or less, 14.6 µm or less, 14 µm or less, 13.5 µm or less, 13 µm or less, 12.5 µm or less or 12.4 µm or less. Especially, the dissolution rate of active ingredients may be superior when the D90 is within the above-described range.

In an exemplary embodiment of the present disclosure, the ginseng powder may comprise particles, wherein the particles have a D50 of 3-8 µm, and wherein the D50 is according to a volume-based particle diameter distribution, measured by laser diffractometry. Specifically, the D50 may be more than 2 µm, 2.5 µm or more, 3 µm or more, 3.5 µm or more, 4 µm or more, 4.5 µm or more, 4.6 µm or more, 5 µm or more, 5.5 µm or more or 5.7 µm or more; and less than 8 µm, 7.7 µm or less, 7.5 µm or less, 7 µm or less, 6.5 µm or less, 6 µm or less or 5.7 µm or less. When the D50 is more than the upper limit, the dissolution rate of active ingredients may decrease. And, when the D50 is less than the lower limit, the applicability of the ginseng powder becomes unsatisfactory due to excessively increased sebaceous absorption.

In an exemplary embodiment of the present disclosure, the ginseng powder may comprise particles, wherein the particles have a specific surface area of 1.86-2.74 m²/g determined by the BET method. The specific surface area may be measured by the BET method using nitrogen adsorption according to ISO 9277. The BET method measures specific surface area by adsorbing nitrogen gas onto sample surface and calculating specific surface area using the BET multi-point method. The specific surface area may also be measured by known specific surface area measurement methods. Specifically, the specific surface area may be 1.86 m²/g or more, 1.90 m²/g or more, 1.94 m²/g or more, 1.98 m²/g or more, 2.02 m²/g or more, 2.06 m²/g or more, 2.10 m²/g or more, 2.14 m²/g or more, 2.18 m²/g or more, 2.22 m²/g or more, 2.26 m²/g or more or 2.30 m²/g or more; and 2.74 m²/g or less, 2.70 m²/g or less, 2.66 m²/g or less, 2.62 m²/g or less, 2.58 m²/g or less, 2.54 m²/g or less, 2.50 m²/g or less, 2.46 m²/g or less, 2.42 m²/g or less, 2.38 m²/g or less, 2.34 m²/g or less or 2.30 m²/g or less.

In an exemplary embodiment of the present disclosure, the ginseng powder may be obtained from a tissue-softened ginseng whose surface tissue has been softened and dried, particularly from a tissue-softened fresh ginseng. The tissue softening refers to removing water from ginseng, fresh ginseng, etc. through reduced pressure vacuum and softening the surface tissue.

The inventors of the present disclosure have identified that effective ginseng polysaccharides are contained in ginseng residue which is discarded after extraction of ginseng, and have completed the present disclosure in order to provide a ginseng powder containing the effective ginseng polysaccharide ingredients without an extraction process using a solvent. More specifically, the inventors of the present disclosure have completed the present disclosure in order to utilize ginseng saponins, i.e., ginsenosides, which are major pharmacologically active substances contained in the ginseng residue.

The chemical structures of about 37 ginsenosides have been identified and they have been identified through basic researches to have various pharmacological activities of anti-stress, brain cell-protecting, anti-thrombotic, lipid metabolism-improving, cancer cell proliferation-inhibiting, antidiabetic and anti-fatigue effects [Arch. Pharm. Res. 2000, Oct, 23(5) 518-524, Biochem Pharmacol. 2003 Dec 1; 66(11): 2213-21. Chin J Physiol. 2003 Mar 31; 46(1):1-7., Shibata et al. J. Korean Med Sci. 16 (suppl.) S28-37 (2001)].

Ginsenosides are neutral bisdesmoside glycosides wherein sugar molecules such as glucose, arabinose, xylose, rhamnose, etc. are bound to a triterpenoid-based dammarane backbone. They are classified into panaxadiol-, panaxatriol- and oleanane-based ginsenosides depending on the positions where the sugar molecules are attached and the shape of the backbone structure, and they show different pharmacological activities in the body. In particular, it is reported that the panaxatriol-based ginsenosides have blood pressure-raising, body temperature-elevating and central nervous system-exciting effects, whereas the panaxadiol-based ginsenosides have blood pressure-lowering, body temperature-lowering and nervous system-relaxing effects [Saito et al. Jap. Pham. 22:245-259 (1972)]. The representative examples of the panaxatriol-based ginsenosides are ginsenosides Rg1 and Re, and the representative examples of the panaxadiol-based ginsenosides are ginsenosides Rb1 and Rd.

The inventors of the present disclosure have identified that ginsenosides exhibiting pharmacological activity and physiological activity are contained in ginseng and that, when a tissue-softened ginseng powder containing such ginsenosides is applied to skin, they are dissolved well at the skin temperature without foreign body sensation, and have completed the present disclosure.

In an exemplary embodiment of the present disclosure, the tissue-softened ginseng may be a water-removed dried ginseng. The dried ginseng refers to a ginseng obtained by removing water from fresh ginseng, white ginseng, etc. through reduced pressure vacuum and softening the surface tissue. The tissue of fresh ginseng or white ginseng is softened by removing water. In particular, the inventors of the present disclosure have identified that a ginseng powder derived from dried ginseng has D90 according to a volume-based particle diameter distribution measured by laser diffractometry, of less than 16 µm, and have completed the present disclosure.

In another aspect, the present disclosure provides a cosmetic composition comprising the ginseng powder.

In an exemplary embodiment of the present disclosure, the ginseng powder is contained in an amount of 0.001-100 wt% based on the total weight of the cosmetic composition. Specifically, it may be contained in an amount of 0.001 wt% or more, 0.002 wt% or more, 0.005 wt% or more, 0.007 wt% or more, 0.01 wt% or more, 0.02 wt% or more, 0.05 wt% or more, 0.07 wt% or more, 0.1 wt% or more, 0.2 wt% or more, 0.5 wt% or more, 0.7 wt% or more, 1 wt% or more or 2 wt% or more; and 100 wt% or less, 90 wt% or less, 80 wt% or less, 70 wt% or less, 60 wt% or less, 50 wt% or less, 40 wt% or less, 30 wt% or less, 20 wt% or less, 10 wt% or less, 7 wt% or less, 5 wt% or less or 2 wt% or less.

In an aspect, the cosmetic composition of the present disclosure may be a formulation in the form of a water-in-oil suspension, an oil-in-water suspension, an emulsion, a paste, a gel, a cream, a lotion, a powder, an oil, a stick, a balm or a spray, although not being limited thereto. Each formulation of the composition may contain various ingredients mixed in general compositions depending on the particular formulation or the purpose of use, the type and amount of which may be easily selected by those skilled in the art.

In an exemplary embodiment of the present disclosure, the cosmetic composition may be in the form of a powder formulation since the ginseng powder of the present disclosure has superior dissolution rate. When the ginseng powder of the present disclosure is comprised in a cosmetic composition in the form of a powder formulation, it may further comprise a binder phase, and the binder phase may be a liquid binder phase and/or a solid binder phase. The liquid binder phase may comprise at least one nonvolatile oil that may be selected from a group consisting of a nonvolatile hydrocarbon-based oil and a nonvolatile silicone-based oil. Specifically, the oil may be a natural oil. In an exemplary embodiment of the present disclosure, the natural oil may be a ginseng seed oil. The solid binder phase may comprise one or more selected from a group consisting of a wax and a metallic soap.

In an aspect, the composition of the present disclosure may further comprise, in addition to the ingredients described above, one or more cosmetically acceptable additive, e.g., one or more additive selected from a group consisting of a binder, an excipient, a surfactant, a plasticizer, an antiseptic, a sterilizer, an antioxidant, a metal ion sequestrant, a colorant and a flavorant. In addition, when the ginseng powder of the present disclosure is comprised in a cosmetic composition in powder form, at least one additional ingredient selected from a sunscreen that may be selected from an inorganic sunscreen and an organic sunscreen, an antiseptic, an active cosmetic ingredient, a humectant, a surfactant and/or and an aromatic may be further comprised.

In another aspect, the present disclosure provides a method for preparing the ginseng powder described above, which comprises: a) obtaining a first ginseng powder by coarsely grinding a tissue-softened ginseng; and b) obtaining a second ginseng powder by grinding the first ginseng powder.

In an exemplary embodiment of the present disclosure, the coarse grinding of the a) is grinding the tissue-softened ginseng to a size of about 0.03-0.80 mm and may be performed using a roller-type grinder or a cutter.

In an exemplary embodiment of the present disclosure, the grinding of the b) is for obtaining a ginseng powder comprising particles with D90 and D50 according to a volume-based particle diameter distribution, measured by laser diffractometry, in the range of the present disclosure, and may be performed by dry grinding or wet grinding such as sand milling, pearl milling, rod milling, pin milling, hammer milling, cutter milling, ball milling, jet milling, etc.

In an exemplary embodiment of the present disclosure, the tissue-softened ginseng of the a) may be a water-removed dried ginseng. The dried ginseng refers to a ginseng obtained by removing water from fresh ginseng, white ginseng, etc. through reduced pressure vacuum and softening the surface tissue. The process of removing water may comprise decompressing a ginseng material with a decompressor; and removing water from the ginseng material which has been washed under reduced pressure vacuum condition. In particular, the inventors of the present disclosure have identified that a ginseng powder derived from dried ginseng has D90 according to a volume-based particle diameter distribution, measured by laser diffractometry, of less than 16 µm, and have completed the present disclosure.

In an exemplary embodiment of the present disclosure, the method may further comprise c) of classifying the second ginseng powder. The classifying is not specially limited. For example, the second ginseng powder may be filtered with a filter of 2-8 meshes. Through this, the D50 according to a volume-based particle diameter distribution, measured by laser diffractometry, may be controlled.

### [Examples]

Hereinafter, the present disclosure is described in detail through examples. However, the following examples are provided only as examples for helping understanding of the present disclosure, and the content of the present disclosure is not limited by the examples.

### <Preparation Example>

### 1. Preparation of ginseng powder of Example 1

Washed fresh ginseng was dried through reduced pressure vacuum. A first ginseng powder was obtained by coarsely grinding the dried ginseng using a cutting mill (SM100, Retsch) (cutting speed: 1,500 min⁻¹). Then, a ginseng powder according to Example 1 was obtained by supplying the coarsely ground first ginseng powder to an air jet mill (SD Micronizer, Sturevant) at a speed of 3 g/min and inducing collision of the first ginseng powder using an ultrasonic air flow of 3 g/min.

### 2. Preparation of ginseng powders of Examples 2-5

Ginseng powders according to Examples 2-5 were obtained by classifying the ginseng powder of Example 1 using a filter.

### 3. Preparation of ginseng powders of Comparative Examples 1 and 2

A ginseng powder according to Comparative Example 1 was obtained using ginseng which did not pass through water removal and tissue softening processes, by using a ball mill (PM100, Retsch) instead of an air jet mill. In addition, a ginseng powder according to Comparative Example 2 was obtained by using ginseng which did not go through water removal and tissue softening processes and only by coarsely grinding.

### <Test Example 1> Measurement of particle shape of ginseng powders of Example 1 and Comparative Example 1

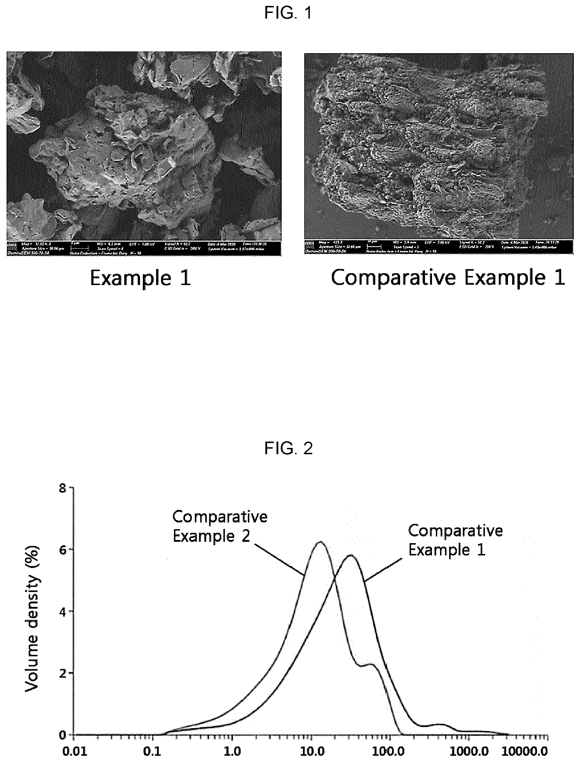

The particle shape of the ginseng powders of Example 1 and Comparative Example 1 was measured using a scanning electron microscope. The measurement result is shown in FIG. 1. From FIG. 1, it can be seen that the ginseng powder of Comparative Example 1, which has not passed through a surface softening process by water removal (evaporation), has a more particle size and a harder surface as compared to Example 1.

### <Test Example 2> Analysis of particle size distribution of ginseng powders of Examples 1-5 and Comparative Examples 1-2

### (1) Analysis of particle size distribution

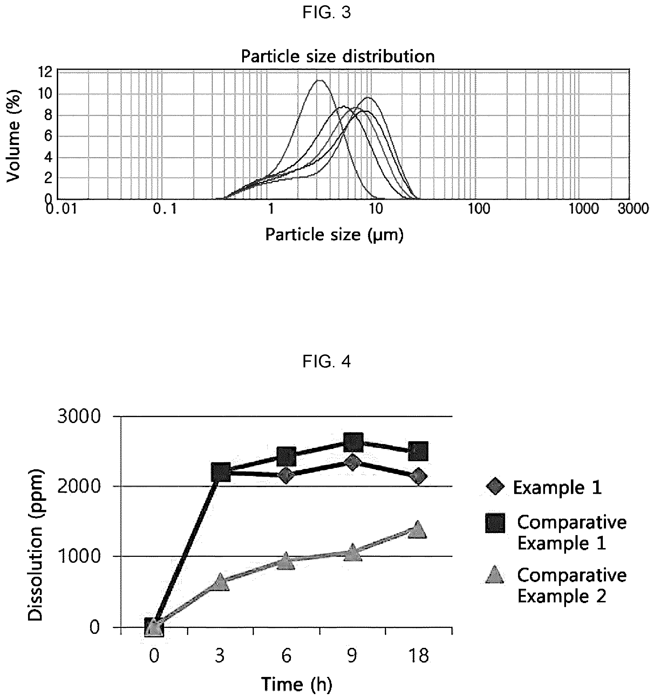

The particle size distribution of the ginseng powders of Examples 1-5 and Comparative Examples 1-2 was measured using a particle size analyzer (Mastersizet 2000, Malvern Panalytical). The result is shown in Table 1, FIG. 2 and FIG. 3.

**[Table 1]**

| | Ex. 1 | Ex. 2 | Ex. 3 | Ex. 4 | Ex. 5 | Comp. Ex. 1 | Comp. Ex. 2 |
|---|---|---|---|---|---|---|---|
| D50 (µm) | 7.7 | 6.5 | 5.7 | 4.6 | 3.0 | 11.4 | 30 |
| D90 (µm) | 15.6 | 14.6 | 12.4 | 10.1 | 5.7 | 49.2 | 92.4 |

### (2) Result of particle size distribution analysis

As can be seen from Table 1, the ginseng powders of Examples 1-5 had D50 and D90 according to a volume-based particle diameter distribution within the range of the present disclosure unlike Comparative Examples 1-2. In particular, as can be seen from FIG. 2, the ginseng powders of Comparative Examples 1-2, which did not pass through the tissue softening process, exhibited D90 exceeding 49 µm. In addition, as can be seen from the result for Comparative Example 2 in FIG. 2, the characteristic peak did not disappear when the tissue softening process was absent.

### <Test Example 3> Analysis of dissolution rate of ginseng powders of Example 1 and Comparative Examples 1-2

### (1) Analysis of dissolution rate

For investigation of the dissolution rate of the ginseng powders of Example 1 and Comparative Examples 1-2, 20 g of each ginseng powder was added to a 200-mL Erlenmeyer flask and the dissolution rate of ginsenosides Rg1, Re, Rb1 and Rd was measured by HPLC for 18 hours. The experiment was performed at temperatures of 31 °C and 70 °C. The result is shown in Table 2 and FIG. 4.

**[Table 2]**

| Dissolution (ppm) | Saponin | 3 hours | 6 hours | 9 hours | 18 hours |
|---|---|---|---|---|---|
| Example 1 (31 °C) | Re | 386.50 | 391.71 | 391.87 | 376.21 |
| | Rg1 | 194.21 | 193.24 | 200.64 | 186.29 |
| | Rb1 | 1559.75 | 1524.17 | 1670.27 | 1520.01 |
| | Rd | 65.35 | 65.28 | 81.54 | 63.18 |
| | Total | 2205.81 | 2174.40 | 2344.32 | 2145.68 |
| Comparative Example 1 (70 °C) | Re | 385.31 | 395.86 | 395.86 | 328.79 |
| | Rg1 | 192.35 | 200.65 | 212.25 | 180.71 |
| | Rb1 | 1567.95 | 1764.15 | 1928.37 | 1913.95 |
| | Rd | 65.07 | 76.64 | 101.92 | 79.70 |
| | Total | 2210.69 | 2437.30 | 2638.40 | 2503.14 |
| Comparative Example 2 (70 °C) | Re | 127.43 | 168.49 | 189.40 | 259.82 |
| | Rg1 | 86.94 | 119.37 | 136.39 | 202.88 |
| | Rb1 | 426.54 | 638.47 | 730.39 | 942.32 |
| | Rd | 4.41 | 13.81 | 16.28 | 20.77 |
| | Total | 645.32 | 940.14 | 1072.46 | 1425.79 |

### (2) Result of dissolution rate analysis

As can be seen from Table 2 and FIG. 4, whereas the initial dissolution (at 3 hours) of the ginseng powder of Comparative Example 1 was 2210.69 ppm at 70 °C, the initial dissolution (at 3 hours) of the ginseng powder of Example 1 was 2205.81 ppm even at the skin temperature of 31 °C. Accordingly, it was confirmed that Example 1 exhibited superior initial dissolution rate even at lower low temperature. Meanwhile, for Comparative Example 2, the initial dissolution rate was very low as 645.32 ppm at 70 °C. The dissolution at 18 hours was 1425.8 ppm, which was also very low as compared to the initial dissolution of Example 1.

### <Test Example 4> Assessment of active ingredient-delivering ability depending on formulation of cosmetic composition

### (1) Preparation of Formulation Example 1 and Formulation Example 2

A gel-type cosmetic composition containing the ginseng powder of Example 1 (Formulation Example 1) and a powder-type cosmetic composition containing the ginseng powder of Example 1 (Formulation Example 2) were prepared.

### (2) Assessment of active ingredient-delivering ability depending on formulation of cosmetic composition

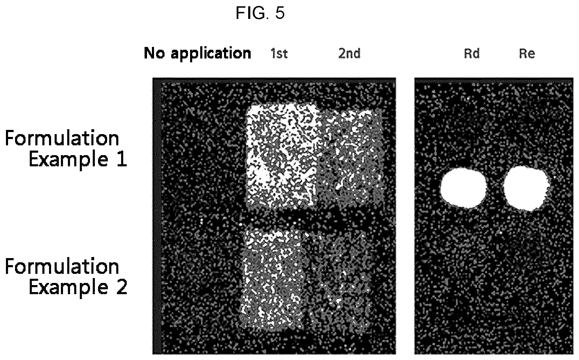

The active ingredient-delivering ability depending on the formulation of the cosmetic composition was assessed by desorption electrospray ionization-mass spectrometry (DESI-MS). First, after applying each composition on skin and waiting for 3 hours, the composition remaining on the skin was wiped out and samples were prepared by taping with 10 sheets of stripping tapes (D-Squame Stripping Discs D101, CuDerm). The transdermal delivery ability was investigated by imaging the active ingredients, ginsenosides Re and Rd, attached to the tapes by DESI-MS (Q-time-of-flight mass spectrometer, QTOF-MS) imaging. The result is shown in FIG. 5. In FIG. 5, the active ingredients are shown with light yellow color.

### (3) Result of active ingredient-delivering ability depending on formulation of cosmetic composition

As can be seen from FIG. 5, the gel-type cosmetic composition of Formulation Example 1 had superior transdermal delivery ability and the powder-type cosmetic composition of Formulation Example 2 also showed superior transdermal delivery ability. Since the ginseng powder according to an exemplary embodiment of the present disclosure exhibits superior dissolution rate as demonstrated in Test Example 2, it exhibits superior availability of active ingredients even when formulated as a powder form.

Hereinafter, the formulation examples of a cosmetic composition comprising the ginseng powder according to the present disclosure are described in more detail. However, other formulation examples are also possible and the scope of the present disclosure is not limited by them.

### [Formulation Example 1] Gel

A gel containing the ginseng powder of Example 1 was prepared as described in Table 3.

**[Table 3]**

| Ingredients | Contents (wt%) |
|---|---|
| Example 1 | 2.0 |
| Sodium ethylenediaminetetraacetate | 0.05 |
| Glycerin | 5.0 |
| Carboxyvinyl polymer | 0.3 |
| Ethanol | 5.0 |
| PEG 60 hydrogenated castor oil | 0.5 |
| Triethanolamine | 0.3 |
| Antiseptic, colorant and flavorant | Adequate |
| Purified water | To 100 |

### [Formulation Example 2] powder

A powder containing the ginseng powder of Example 1 was prepared as described in Table 4.

**[Table 4]**

| Ingredients | Contents (wt%) |
|---|---|
| Example 1 | 2.0 |
| Nylon powder | 5.0 |
| Modified starch | 3.0 |
| Titanium dioxide | 3.0 |
| Mica | 25.0 |
| Squalane | 0.5 |
| Dimethicone | 0.8 |
| Antiseptic, colorant and flavorant | Adequate |
| Silicone-treated talc | To 100 |

### [Formulation Example 3] Nourishing cream

A nourishing cream containing the ginseng powder of Example 1 was prepared as described in Table 5.

**[Table 5]**

| Ingredients | Contents (wt%) |
|---|---|
| Example 1 | 2.0 |
| Polysorbate 60 | 1.5 |
| Sorbitan sesquioleate | 0.5 |
| PEG 60 hydrogenated castor oil | 2.0 |
| Liquid paraffin | 10 |
| Squalane | 5.0 |
| Caprylic/capric triglyceride | 5.0 |
| Glycerin | 5.0 |
| Butylene glycol | 3.0 |
| Propylene glycol | 3.0 |
| Triethanolamine | 0.2 |
| Antiseptic, colorant and flavorant | Adequate |
| Purified water | To 100 |

### [Formulation Example 4] Pack

A pack containing the ginseng powder of Example 1 was prepared as described in Table 6.

**[Table 6]**

| Ingredients | Contents (wt%) |
|---|---|
| Example 1 | 1.0 |
| Polyvinyl alcohol | 13.0 |
| Sodium carboxymethyl cellulose | 0.2 |
| Glycerin | 5.0 |
| Allantoin | 0.1 |
| Ethanol | 6.0 |
| PEG 12 nonyl phenyl ether | 0.3 |
| Polysorbate 60 | 0.3 |
| Antiseptic, colorant and flavorant | Adequate |
| Purified water | To 100 |

The present disclosure relates to and includes at least the following aspect s.
[Aspect 1] A ginseng powder comprising particles, wherein the particles have a D90 less than 16 µm, and wherein the D90 is according to a volume-based particle diameter distribution, measured by laser diffractometry.
[Aspect 2] The ginseng powder according to the aspect 1, wherein the particles have a D50 of 3-8 µm, and wherein the D50 is according to a volume-based particle diameter distribution, measured by laser diffractometry.
[Aspect 3] The ginseng powder according to any of the aspects 1 and 2, wherein the particles have a specific surface area of 1.86-2.74 m²/g determined by the BET method.
[Aspect 4] The ginseng powder according to any of the aspects 1 to 3, wherein the ginseng powder is obtained from a tissue-softened ginseng.
[Aspect 5] The ginseng powder according to any of the aspects 1 to 4, wherein the tissue-softened ginseng is a water-removed ginseng.
[Aspect 6] A cosmetic composition containing the ginseng powder according to any of the aspects 1 to 5.
[Aspect 7] The cosmetic composition according to the aspect 6, wherein the ginseng powder is contained in an amount of 0.001 -100 wt% based on the total weight of the cosmetic composition.
[Aspect 8] The cosmetic composition according to any of the aspects 6 and 7, wherein the formulation of the cosmetic composition is in powder form.
[Aspect 9] A method for preparing the ginseng powder according to any of the aspects, comprisings: a) obtaining a first ginseng powder by coarsely grinding a tissue-softened ginseng; and b) obtaining a second ginseng powder by grinding the first ginseng powder.
[Aspect 10] The method for preparing a ginseng powder according to the aspect 9, wherein the tissue-softened ginseng of the a) is a water-removed ginseng.
[Aspect 11] The method for preparing a ginseng powder according to any of the aspects 9 and 10, which further comprises: c) classifying the second ginseng powder.

While the present disclosure has been described with reference to the specific exemplary embodiments, various changes or modifications can be made without departing from the subject matter and scope of the present disclosure. Accordingly, such changes or modifications encompassed in the subject matter of the present disclosure will be included within the appended claims.

## Claims

1. A ginseng powder comprising particles, wherein the particles have a D90 less than 16 µm, and wherein the D90 is according to a volume-based particle diameter distribution measured by laser diffractometry.

2. The ginseng powder according to claim 1, wherein the particles have a D50 of 3-8 µm, and wherein the D50 is according to a volume-based particle diameter distribution measured by laser diffractometry.

3. The ginseng powder according to any of claims 1 and 2, wherein the particles have a specific surface area of 1.86-2.74 m²/g determined by the BET method.

4. The ginseng powder according to any of claims 1 to 3, wherein the ginseng powder is obtained from a tissue-softened ginseng.

5. The ginseng powder according to claim 4, wherein the tissue-softened ginseng is a water-removed ginseng.

6. A cosmetic composition comprising the ginseng powder according to any of claims 1 to 5.

7. The cosmetic composition according to claim 6, wherein the ginseng powder is comprised in an amount of 0.001-100 wt% based on the total weight of the cosmetic composition.

8. The cosmetic composition according to any of claims 6 and 7, wherein the formulation of the cosmetic composition is in powder form.

9. A method for preparing the ginseng powder according to any of claims 1 to 5, comprising:
a) obtaining a first ginseng powder by coarsely grinding a tissue-softened ginseng; and
b) obtaining a second ginseng powder by grinding the first ginseng powder.

10. The method for preparing a ginseng powder according to claim 9, wherein the tissue-softened ginseng of the a) is a water-removed ginseng.

11. The method for preparing a ginseng powder according to any of claims 9 and 10, which further comprises: c) classifying the second ginseng powder.
